# EUROPÄISCHE PATENTANMELDUNG

(11) **EP 0 820 753 A2**
(43) Veröffentlichungstag der Anmeldung: **28.01.1998**
(21) Anmeldenummer: 97112548.9
(22) Anmeldetag: 22.07.1997
(51) Int. Cl.: A61J 3/06

(54) **Verfahren zur Herstellung von festen Arzneiformen**

(30) Priorität: 23.07.1996 DE 19629753
(71) Anmelder: BASF AKTIENGESELLSCHAFT, 67056 Ludwigshafen (DE)
(72) Erfinder: Zettler, Hans Dieter, 67269 Grünstadt (DE); Schiessl. Michael, 67454 Hassloch (DE); Breitenbach, Jörg, Dr., 68199 Mannheim (DE); Rosenberg, Jörg, Dr., 67158 Ellerstadt (DE)
(74) Vertreter: Kinzebach, Werner, Dr.

(57) **Zusammenfassung**

Die Erfindung betrifft ein Verfahren zur Herstellung von festen Arzneiformen durch Vermischen und Aufschmelzen von mindestens einem pharmakologisch akzeptablen polymeren Bindemittel, mindestens einem pharmazeutischen Wirkstoff und gegebenenfalls üblichen pharmazeutischen Additiven in Abwesenheit eines Lösungsmittels zu einem plastischen Gemisch und Ausformen des Gemisches zu der gewünschten Arzneiform durch Extrusion, wobei das Ausformen in zwei Schritten erfolgt, wobei man in einem ersten Schritt den extrudierten Strang zu Formkörpern zerteilt und diese Formkörper in einem zweiten Schritt in plastischem Zustand arrondiert.

## Beschreibung

Die vorliegende Erfindung betrifft ein Verfahren zur Herstellung von festen Arzneiformen durch Vermischen und Aufschmelzen von mindestens einem pharmakologisch akzeptablen polymeren Bindemittel, mindestens einem pharmazeutischen Wirkstoff und gegebenenfalls üblichen pharmazeutischen Additiven in Abwesenheit eines Lösungsmittels zu einem plastischen Gemisch und Ausformen des Gemisches zu der gewünschten Arzneiform.

Die klassischen Verfahren zur Herstellung fester Arzneiformen, insbesondere Tabletten, werden diskontinuierlich durchgeführt und umfassen mehrere Stufen, Pharmazeutische Granulate stellen hierbei ein wichtiges Zwischenprodukt dar. So ist z.B. dem Buch "Pharmazeutische Technologie", Verfasser Profn. Bauer, Frömmig und Führer, Thieme Verlag, Seiten 292 ff, zu entnehmen, daß man Arzneiformen über Trockengranulierung aus der Schmelze gewinnen kann. Es wird beschrieben, daß Schmelzerstarrungsgranulate entweder durch Schmelzen und Schockerstarren, durch Ausgießen und Zerkleinern oder durch Sprüherstarren in Sprühtürmen hergestellt werden können. Ein Problem bei diesen Verfahren ist die für die Herstellung von Arzneimitteln erforderliche exakte Formgebung. Es werden häufig unregelmäßige Partikel oder Bruchstücke erzeugt, so daß die erzielte Form in keiner Weise den üblichen Arzneiformen entspricht und Granulate deshalb als eigenständige Arzneiform nur eine geringe Bedeutung besitzen. Die Herstellung der gewünschten festen Arzneiformen erfordert den Einsatz weiterer Verfahrensschritte, wie zum Beispiel die Komprimierung mit Hilfe von Tablettenmaschinen. Dies ist zeit- und kostenintensiv.

Seit einiger Zeit ist ein wesentlich einfacheres kontinuierliches Verfahren zur Herstellung fester Arzneiformen bekannt, bei dem man eine wirkstoffhaltige lösungsmittelfreie Schmelze aus einem polymeren wirkstoffhaltigen Bindemittel extrudiert und den extrudierten Strang zu der gewünschten Arzneiform formt, beispielsweise in einem Kalander mit Formwalzen, siehe EP-A-240 904, EP-A-240 906 und EP-A-337 256 und EP 358105. Obwohl so eine gezielte Formgebung erzielt werden kann, ist ein wesentlicher Nachteil dieses Verfahrens, daß sich zwischen den Tablettenkörpern Schwimmhäute bilden oder die Tabletten Grate aufweisen, von denen sie in anschließenden Verfahrensschritten befreit werden müssen. Solche Verfahren sind aufwendig, führen in vielen Fällen zu Produktverlusten in Form von beschädigten Tabletten, die aus verschiedenen Gründen nicht immer in den Prozeß zurückgeführt werden können.

Der vorliegenden Erfindung liegt daher die Aufgabe zugrunde, ein einfaches und kostengünstiges Verfahren zur Herstellung von festen Arzneiformen, insbesondere von Tabletten mit definierter Form, zur Verfügung zu stellen.

Überraschenderweise wurde nun gefunden, daß diese Aufgabe gelöst wird, wenn das Ausformen des extrudierten plastischen Gemisches in zwei separaten Schritten durchgeführt wird, nämlich Schneiden des extrudierten Stranges und Arrondieren der erhaltenen Stücke in plastischem Zustand.

Gegenstand der vorliegenden Erfindung ist daher ein Verfahren zur Herstellung von festen Arzneiformen durch Vermischen und Aufschmelzen von mindestens einem pharmakologisch akzeptablen polymeren Bindemittel, mindestens einem pharmazeutischen Wirkstoff und gegebenenfalls üblichen pharmazeutischen Additiven in Abwesenheit eines Lösungsmittels zu einem plastischen Gemisch und Ausformen des Gemisches zu der gewünschten Arzneiform durch Extrusion, das dadurch gekennzeichnet ist, daß das Ausformen in zwei Schritten erfolgt, wobei man in einem ersten Schritt den extrudierten Strang zu Formkörpern zerteilt und diese Formkörper in einem zweiten Schritt in plastischem Zustand arrondiert.

Erfindungsgemäß wird das plastische Gemisch zunächst unter Verwendung eines geeigneten Extrusionswerkzeuges zu einem fortlaufenden Strang extrudiert. Die Form des Extrusionswerkzeuges richtet sich nach der gewünschten Arzneiform. Beispielsweise sind Rundlochwerkzeuge oder Coextrusionswerkzeuge, wie Werkzeuge mit kreisring-spaltförmigen Austrittsquerschnitt geeignet. Coextrusionswerkzeuge dienen zur Herstellung mindestens zweischichtiger, offener oder geschlossener Arzneiformen. Eine der Schichten enthält dabei einen Wirkstoff, die andere Schicht kann wirkstofffrei sein oder gegebenenfalls einen anderen Wirkstoff enthalten. Weitere Angaben sind der DE 195 39 361.9 zu entnehmen.

Vorzugsweise werden jedoch Rundlochdüsen verwendet, wobei das plastische Gemisch als Strang mit kreisförmigem Querschnitt ausgeformt und ausgetragen wird.

Die Düsenauslegung erfolgt in Abhängigkeit von dem zur Anwendung kommenden polymeren Bindemittel und der gewünschten Arzneiform. Vorzugsweise verden die Düsen auf einer Linie waagrecht angeordnet, wobei die Abstände der Bohrungen im allgemeinen einem Mehrfachen ihres Durchmessers entsprechen. Nach dem Austrag aus dem Extrusionswerkzeug erfolgt die Zerlegung bzw. Zerteilung des Stranges, vorzugsweise durch Zerschneiden bzw. Zerhacken (Kaltabschlag). Der Zeitpunkt der Zerteilung erfolgt in Abhängigkeit von der Geschwindigkeit des austretenden Stranges und der gewünschten Arzneiform. Es ist dabei von Vorteil, die Stränge nach dem Austritt aus der Düse mechanisch zu unterstützen, z.B. durch eine waagrechte Platte oder ein umlaufendes Band. Man erhält beispielsweise Zylinder mit einer definierten Länge L und einem definierten Durchmesser D. Das Verhältnis L/D kann je nach gewünschter Arzneiform variieren. Wenn das Verhältnis >1 ist (z.B. ≥2) erhält man Oblongtabletten, wenn es ≤1 ist, kann man kugelförmige Tabletten erhalten.

Die Zerlegvorrichtung wird vorzugsweise für jeden Strang separat gesteuert. Die gewünschte Länge des Formkörpers kann z.B. durch ein optisches System gemessen werden. Bei Erreichen der gewünschten Länge (Abstand bis zur Lochplatte) gibt dieses optische System ein Signal an eine Schneidvorrichtung ab, die unabhängig von den anderen, jeweils einer Bohrung zugeordneten Schneidvorrichtungen, den Strang abschneidet und wieder in ihre Ausgangsposition zurückschwenkt.

Die erhaltenen Formkörper werden erfindungsgemäß in einem zweiten Schritt arrondiert. Unter Arrondieren ist hier ein Runden der Kanten und Ecken des Formkörpers zu verstehen, ohne daß sich dessen Masse wesentlich ändert. Das Arrondieren erfolgt durch Kontakt des Formkörpers mit einem oder mehreren Arrondierwerkzeugen. Dabei vollzieht entweder der Formkörper oder das (die) Arrondierwerkzeug(e) eine Bewegung, während der jeweils andere ruht. In bestimmten Fällen kann es auch von Vorteil sein, daß sich beide bewegen, um eine bestimmte Formgebung zu erzielen. Die Bewegung des Formkörpers, z.B. Rollieren, kann beispielsweise dadurch erreicht werden, daß er zwischen eine stehende und eine bewegte Fläche, vorzugsweise zwei Platten oder eine Platte und ein bewegtes Band, gebracht wird. Die Bewegung des Arrondierwerkzeugs kann auf übliche Art und Weise realisiert werden.

Als Arrondierwerkzeug verwendet man vorzugsweise eine gebogene, insbesondere halbkreisförmige Backe, die insbesondere ein metallisches Teil ist, das auf der den zylindrischen Abschnitten zugewandten Seite eine im wesentlichen konkave Form aufweist.

Der Arrondierungsvorgang setzt voraus, daß sich der Formkörper in plastischem Zustand befindet. Das kann dadurch erreicht werden, daß man beispielsweise den Abkühlvorgang des Formkörpers verzögert und/oder die Zeitspanne zwischen dem Austritt des Stranges aus der Düse und dem Arrondieren möglichst kurz wählt. Eine andere Möglichkeit besteht darin, die Temperatur des Arrondierwerkzeugs, z.B. durch Beheizen, so zu regeln, daß bei Kontakt mit dem Formkörper dieser die geeignete Plastizität besitzt.

Nach dem Formvorgang läßt man die Arzneiformen auskühlen und fest werden, z.B. auf einem Kühlband.

Das vorliegende Verfahren zur Herstellung von festen Arzneiformen umfaßt auch das Vermischen und Aufschmelzen von mindestens einem pharmakologisch akzeptablen polymeren Bindemittel, mindestens einem pharmazeutischen Wirkstoff und gegebenenfalls üblichen pharmazeutischen Additiven in Abwesenheit eines Lösungsmittels zu einem plastischen Gemisch. Diese Verfahrensschritte können auf bekannte Art und Weise durchgeführt werden, wie beispielsweise in der EP-A-240 904, EP-A-337 256 und EP-A-358 105 beschrieben wird. Auf den Inhalt dieser Publikationen wird hiermit Bezug genommen.

Die Komponenten können zuerst vermischt und dann aufgeschmolzen und homogenisiert werden. Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen, wobei die Rührkessel, Rührwerke, Feststoffmischer etc. gegebenenfalls im Wechsel betrieben werden, und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) in fester Form oder als Lösung oder Dispersion eingesetzt werden.

Das Aufschmelzen und Vermischen erfolgt in einer für diesen Zweck üblichen Vorrichtung. Besonders geeignet sind Extruder oder beheizbare Behälter mit Rührwerk, z.B. Kneter, (wie der unten noch erwähnten Art).

Als Mischapparat sind auch solche Vorrichtungen brauchbar, die in der Kunststofftechnologie zum Mischen eingesetzt werden.

Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z.B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z.B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z.B. über eine Differentialdosierwaage eingeführt werden. Plastische Massen können direkt aus einem Extruder eingespeist oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Das durch Vermischen und Aufschmelzen des Bindemittels, des Wirkstoffes und gegebenenfalls des Additivs oder der Additive erhaltene Gemisch ist teigig bis zähflüssig (thermoplastisch) und daher auch extrudierbar. Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein. Beispiele für geeignete Bindemittel sind:

Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Die K-Werte (nach H. Fikentscher, Cellulose-Chemie 13 (1932), Seiten 58 bis 64 und 71 und 74) der Polymere liegen im Bereich von 10 bis 100, vorzugsweise 12 bis 70, insbesondere 12 bis 35, für PVP > 17, insbesondere 20 bis 35.

Bevorzugte polymere Bindemittel sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen.

Das polymere Bindemittel muß in der Gesamtmischung aller Komponenten im Bereich von 50 bis 180°C, vorzugsweise 60 bis 130°C erweichen oder schmelzen. Die Glasübergangstemperatur der Mischung muß daher unter 180°C, vorzugsweise unter 130°C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluß zeigen. Vorzugsweise aber enthält das Gemisch keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-% bezogen auf das Polymerisat, betragen kann, sind z.B.
Streckmittel bzw. Füllstoffe, wie Silikate oder Kieselerde, Magnesiumoxid, Aluminiumoxid, Titanoxid, Stearinsäure oder deren Salze, z.B. das Magnesium- oder Kalziumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches.
Schmiermittel, wie Aluminium- und Calciumstearat, Talcum und Silicone, in einer Konzentration von 0,1 bis 5, vorzugsweise 0,1 bis 3 Gew.-%, bezogen auf das Gesamtgewicht des Gemisches.
Fließmittel, wie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht;
Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;
Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z.B. H. Sucker et al. Pharmazeutische Technologie, Thieme-Verlag, Stuttgart 1978).

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrit-tetracaetat, Polymere wie z.B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z.B. bei J. L. Ford, Pharm. Acta Helv. 61, 69-88 (1986) angegeben.

Als pharmazeutische Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe, sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z.B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika.

Das erfindungsgemäße Verfahren ist beispielsweise zur Verarbeitung folgender Wirkstoffe geeignet:
Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, doxocyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labatalon, Lactulose, Lecithin, Levocarnitin, Levodopa, Levoglutamid, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. - Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Promocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Volinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Im einzelnen kann es zur Ausbildung von festen Lösungen kommen. Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Das erhaltene Gemisch ist lösungsmittelfrei, d.h. es enthält weder Wasser noch ein organisches Lösungsmittel.

Mit dem erfindungsgemäßen Verfahren herstellbare feste pharmazeutische Formen sind insbesondere Tabletten, vorzugsweise Oblongtabletten, Dragees, Pastillen und Pellets. Die erhaltenen Arzneiformen können abschließend auch in üblicher Weise mit Filmüberzügen versehen werden, welche die Wirkstofffreisetzung kontrollieren oder den Geschmack abdecken. Geeignete Materialien für derartige Überzüge sind Polyacrylate, wie die Eudragit-Typen, Celluloseester, wie die Hydroxypropylmethylcellulosephthalate, sowie Celluloseether, wie Ethylcellulose, Hydroxypropylmethylcellulose oder Hydroxypropylcellulose.

Mit dem erfindungsgemäßen Verfahren ist es somit möglich, besonders exakt dimensionierte Arzneiformen herzustellen. Überraschenderweise ist dieses Verfahren preiswert, läßt sehr große Stückzahlen pro Zeiteinheit erzielen und vermeidet jeglichen Abfall.

Die nachfolgenden Beispiele erläutern die Erfindung ohne sie zu begrenzen. Die Figuren 1 bis 7 beziehen sich auf die Beispiele und werden im folgenden kurz beschreiben.

In der Zeichnung zeigt:
- Figur 1: eine Extrudiervorrichtung zur Durchführung des ersten Schritts des erfindungsgemäßen Verfahrens im Längsschnitt;
- Figur 2: eine Variante der Extrudiervorrichtung der Figur 1;
- Figur 3: einen Sammler für die extrudierten Formkörper;
- Figur 4: eine erste Ausführungsform eines Arrondierwerkzeugs zur Durchführung des zweiten Schritts des erfindungsgemäßen Verfahrens im Querschnitt;
- Figur 5: einen Längsschnitt des Arrondierwerkzeugs der Figur 4 entlang der Linie V-V;
- Figur 6: eine Aufsicht auf eine zweite Ausführungsform des Arrondierwerkzeugs zur Durchführung des zweiten Schritts des erfindungsgemäßen Verfahrens;
- Figur 7: das Arrondierwerkzeug der Figur 6 im Querschnitt entlang der Linie VII-VII.

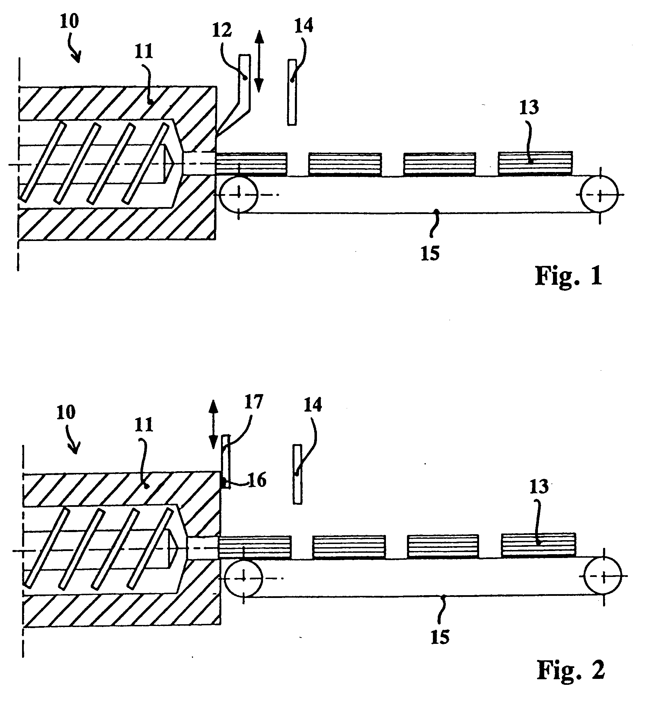

In Figur 1 ist ein Schneckenextruder 10 dargestellt, der über Düsen 11 eine plastische Masse in Form von Produktsträngen extrudiert, die von einem umlaufenden Förderband 15 aufgenommen werden. Eine am Austritt der Düsen 11 angeordnete Schneidvorrichtung, im Fall der Extrudiervorrichtung der Figur 1 ein Messer 12, zerteilt die extrudierten Produktstränge in zylindrische Formkörper 13. Die Länge der Formkörper wird durch einen über jeder Strangbahn angeordneten Sensor 14 bestimmt, dessen Abstand von dem Messer 12 der Länge des zylindrischen Formkörpers 13 entspricht.

Die in Figur 2 dargestellte Ausführungsform der Extrudiervorrichtung unterscheidet sich von der Ausführungsform der Figur 1 im wesentlichen dadurch, daß als Schneidvorrichtung anstelle des Messers 12 ein gegebenenfalls beheizbarer Draht 16 vorgesehen ist, der, in einem Drahthalter 17 angeordnet, auf- und abbewegbar ist.

Das Messer 12 der Vorrichtung der Figur 1 und der Schneidedraht 16 der Vorrichtung der Figur 2 werden über den Sensor 14 gesteuert. Der Sensor 14 registriert das vordere Ende eines Produktstranges und gibt daraufhin einen Steuerimpuls zur Schneidvorrichtung, die das Messer 12 bzw. den Draht 16 in Bewegung setzt, um die hintere Schnittfläche des zylindrischen Formkörpers 13 zu erzeugen.

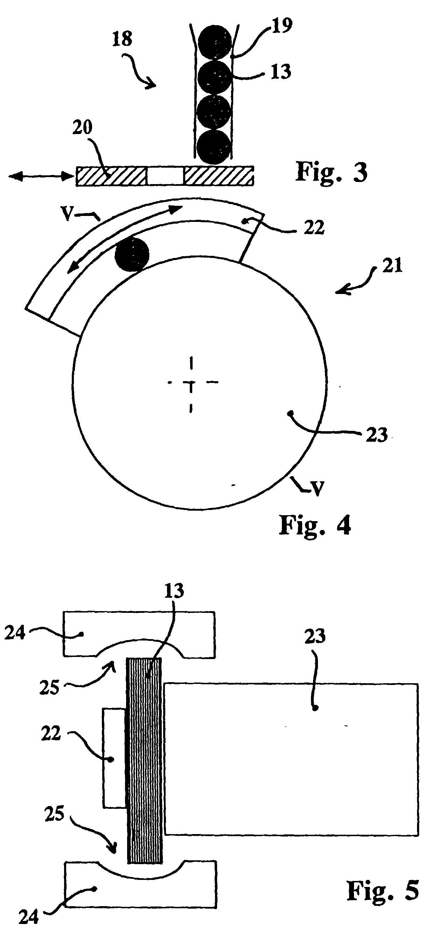

In Figur 3 ist ein Sammler 18 dargestellt, in welchen die extrudierten und auf Länge geschnittenen Formkörper 13 eingebracht werden. Der Sammler 18 kann als Sammelbehälter mit einer oberen trichterförmigen Einfüllöffnung und einer unteren Ausgabeöffnung ausgebildet sein. Bei einer besonders einfachen Ausführungsform des Sammlers 18 kann dieser jedoch auch aus zwei im wesentlichen parallel angeordneten Leitblechen 19 bestehen, deren Abstand voneinander etwas größer als der Durchmesser der zylindrischen Formkörper 13 ist. Die Leitbleche 19 bilden im dargestellten Beispiel eine sich im oberen Bereich trichterförmig erweiternde Einfüllöffnung. Die zylindrischen Formkörper 13 werden in dem Sammler der Figur 3 so angeordnet, daß sie einreihig übereinanderliegen, wobei ihre Längsachsen parallel zu den Leitblechen 19 verlaufen. Die untere Ausgabeöffnung des Sammlers 18 ist im geschlossenen Zustand durch einen Lochschieber 20 verschlossen. Wenn ein einzelner Formkörper 13 ausgegeben werden soll, wird der Lochschieber 20 kurzzeitig so bewegt, daß ein in ihm ausgespartes Langloch, dessen Abmessungen im wesentlichen der Länge und Breite der Formkörper 13 entsprechen, so unter die Öffnung des Sammlers 18 verschoben wird, daß ein einzelner Formkörper durch das Langloch nach unten fallen kann.

Der ausgegebene Formkörper 13 wird einem Arrondierwerkzeug 21 zugeführt.

In den Figuren 4 und 5 ist eine erste Ausführungsform des Arrondierwerkzeugs 21 dargestellt. Bei dieser Ausführungsform besteht das Arrondierwerkzeug aus einem kreisbogenförmigen, beweglichen Mitnehmer 22 und einer stehenden Walze 23. Der Abstand der inneren Mantelfläche des kreisbogenförmigen Mitnehmers 22 von der äußeren Mantelfläche der Walze 23 entspricht gerade dem Durchmesser des zylindrischen Formkörpers 13. Der zu arrondierende Formkörper 13 wird zwischen dem Mitnehmer 22 und der stehender Walze 23 so angeordnet, daß seine Längsachse im wesentlichen parallel zur Drehachse des um das Zentrum der stehenden Walze 23 zumindest in einem Winkelsektor drehbaren kreisbogenförmigen Mitnehmers verläuft. Die innere Mantelfläche des Mitnehmers 22 kommt reibschlüssig auf der äußeren Mantelfläche des zylindrischen Formkörpers 13 in Anlage, so daß der Formkörper 13 bei einer Bewegung des Mitnehmers 22 auf der stehenden Walze 23 abrollt.

In Figur 5 ist das Arrondierwerkzeug 21 in einem Längsschnitt entlang der Linie V-V in Figur 4 dargestellt. Man erkennt, daß in der Nähe der beiden Enden des zylindrischen Formkörpers quaderförmige Backen 24 angeordnet sind, die auf der dem Formkörper 13 zugewandten Fläche eine konkave Ausnehmung 25 aufweisen. Die Backen 24 bestehen bevorzugt aus Metall und können beheizbar sein. Sie sind entlang der Längsachse des zylindrischen Formkörpers bewegbar, wobei die Mittelpunkte der konkaven Ausnehmungen auf dieser Längsachse liegen. Zur Arrondierung werden die quaderförmigen Metallbacken kontinuierlich in Richtung auf die glatten Schnittflächen an den beiden Enden des zylindrischen Formkörpers 13 bewegt, während dieser durch den kreisbogenförmigen Mitnehmer 22 auf der stehenden Walze 23 hinund hergerollt wird. Sobald die Metallbacken den äußeren Rand der Endflächen des Formkörpers 13 berühren, werden diese Schnittflächen nach und nach abgerundet. Die beiden einander gegenüberliegenden Backen 24 nähern sich so lange an, bis die beiden Endflächen des Formkörpers 13 vollständig abgerundet sind.

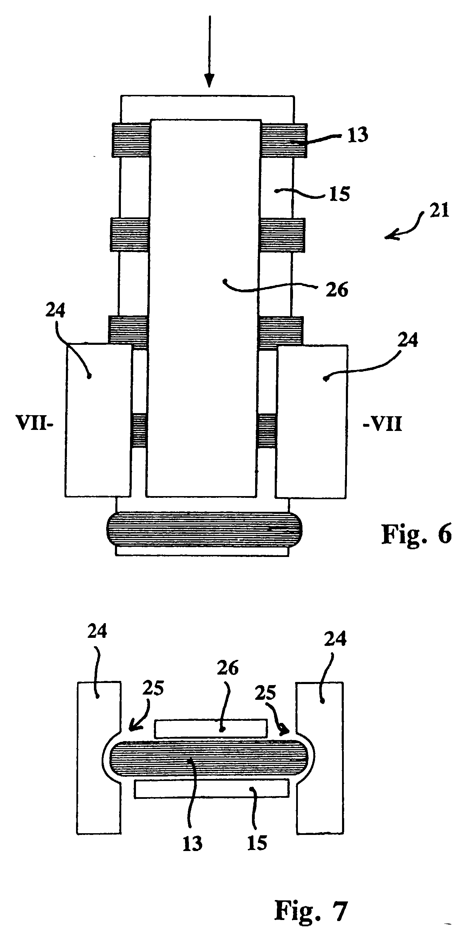

In den Figuren 6 und 7 ist eine zweite Ausführungsform des Arrondierwerkzeugs 21 dargestellt. Die zylindrischen Formkörper 13 werden, wie im Beispiel der Figuren 4 und 5, mit ihren Schnittkanten an beheizten quaderförmigen Backen 24 vorbeigeführt. Die Ausnehmungen der beiden gegenüberliegenden Backen 24 sind im wesentlichen halbzylinderförmig ausgebildet und laufen in Bewegungsrichtung der Formkörper 13 konisch zusammen. Um die extrudierten Formkörper 13 in eine Rollbewegung zu versetzen, werden sie bei der Ausführungsform der Figuren 6 und 7 auf dem Förderband 15, das sie in das Arrondierwerkzeug 21 transportiert, um 90° gedreht, so daß ihre Längsachse quer zur Transportrichtung angeordnet ist. Alternativ kann auch ein zweites (nicht dargestelltes) Förderband eingesetzt werden, das die Formkörper in der gewünschten Orientierung von dem ersten Förderband übernimmt. Über dem Förderband ist eine ruhende Platte 26 angeordnet, die reibschlüssig mit den unter ihr hindurchtransportierten Formkörper 13 in Kontakt kommt und diese in Rotation versetzt. Die Formkörper rollen quasi auf der ruhenden Platte 26 ab und gelangen so in den Eingriffsbereich der quaderförmigen, konisch zusammenlaufenden Arrondierbacken.

Im folgenden wird das erfindungsgemäße Verfahren anhand von zwei Beispielen erläutert.

### Beispiel 1:

### Herstellung von Oblongtabletten unter Verwendung eines Zweischneckenextruders

Eine Polymer/Wirkstoff-Mischung (300 kg Polyvinylpyrrolidon mit K-Wert 30, 6 kg Aerosil 90, 54 kg Maltodextrin, 240 kg Ibuprofen) wird mit einem kämmenden, selbstreinigenden Gleichdrall-Zweischneckenextruder 10 mit einem Schneckendurchmesser von 57 mm zubereitet. Die plastische Masse wird mit einem Durchsatz von 100 kg/h durch zehn auf einer Linie angeordnete Düsen 11 mit einem Durchmesser von jeweils 8 mm extrudiert und von einem umlaufenden Förderband 15 aufgenommen. Das Band bewegt sich mit der Geschwindigkeit des austretenden Stranges. Im vorliegenden Fall beträgt die Geschwindigkeit ca. 0,85 m/min bei einer Strangdichte von
∼ 1g/cm³. Über jeder Strangbahn ist ein Sensor 14 angebracht, dessen Abstand von der Düse der Länge einer Oblongtablette entspricht. Ortet der Sensor den Stranganfang, sendet er einen Impuls an die Schneidvorrichtung und setzt diese in Bewegung, um den Schnitt zu realisieren. Als Schneidwerkzeug kann ein Messer 12 (Figur 1), das nach dem Schnitt sofort wieder in die Ausgangsposition zurückkehrt, oder ein gegebenenfalls beheizbarer Draht 16 (Figur 2), der sowohl bei der Abwärts- als auch bei der Aufwärtsbewegung einen Schnitt ausführen kann, zur Anwendung kommen. Bei einer Oblongtablettenlänge von ca. 2 cm beträgt die Schneidfrequenz ∼50/min.

Die auf dem Band abgekühlten zylindrischen Formkörper 13 werden in einem Sammler 18 (Figur 3) zwischenbevorratet und anschließend über einen Lochschieber 20 einzeln einem Arrondierwerkzeug 21 (Figur 4) zugeführt. Dabei hält und bewegt ein kreisbogenförmiger Mitnehmer 22 einen Formkörper 13 gegen eine stehende Walze 23 und versetzt ihn so in Rotation um seine Längsachse. Während der Formkörper 13 die rotierende Bewegung ausführt, werden dessen glatte Enden an einem beheizten Arrondierwerkzeug vorbeigeführt (Figur 5). Das Arrondierwerkzeug besteht aus paarweise angeordneten Metallbacken 24, die an den Seiten, die dem Formkörper 13 zugewandt sind, konkave Ausnehmungen 25 aufweisen. Die beiden Metallbacken sind entlang der Längsachse der Formkörper 13 bewegbar, wobei die Mittelpunkte der kugelsegmentförmigen Ausnehmungen auf dieser Längsachse liegen. Der Formkörper 13 wird wie vorstehend beschrieben arrondiert, wobei der Kantenumfang des Formkörpers mit fortschreitender Bewegung der Metallbacken kontinierlich abnimmt. Die Bewegung der Metallbacken 24 bricht dann ab, wenn der Umfang der Kanten Null erreicht und infolgedessen die gesamten Querschnittsflächen der arrondierten Formkörper 13 mit zumindest einem Teil der kugelsegmentförmigen Ausnehmung in Kontakt stehen.

Man erhält eine Oblongtablette ohne jeglichen Abfall zu produzieren. Am Ende des Arrondiervorganges wird die Tablette aus dem Prozeß ausgetragen.

Die so erhaltenen Oblongtabletten können einer Nachbehandlung, beispielsweise Beschichtungsvorgängen, unterzogen oder direkt der Verpackung zugeführt werden.

### Beispiel 2:

### Herstellung von Oblongtabletten unter Verwendung eines Ko-Kneters

Anstelle des in Beispiel 1 verwendeten Zweischneckenextruders kommt ein Ko-Kneter mit einem Durchmesser von 70 mm zur Anwendung. Dieser Ko-Kneter bewirkt einen besonders intensiven Mischvorgang, da der Drehbewegung eine hin- und hergehende Bewegung überlagert ist. Die zubereitete plastische Masse wird bei einem Durchsatz von 100 kg/h über eine Zahnradpumpe, die für einen gleichmäßigen Austrag sorgt, und über Düsen ausgetragen. Das Schneiden des Rundstranges erfolgt wie in Beispiel 1 dargestellt. Auf dem Förderband 15 werden die Formkörper 13 um 90^{o} gedreht, so daS sie senkrecht zu ihrer Längsachse zwischen dem Band und einer stehenden Platte 26 eine translatorische Bewegung vollziehen, die der Bewegung des Förderbandes gleichgerichtet ist (Figur 6). Die Formkörper 13 rotieren dabei gleichzeitig um ihre Längsachse. Die Arrondierung der glatten Flächen erfolgt analog zu Beispiel 1, an Arrondierbacken 24, die halbzylindrische, konisch zusammenlaufende Ausnehmungen 25 aufweisen (Figur 7).

Auch hier kann die Oblongtablette nach Beendigung des Arrondiervorganges einem anschließenden Beschichtungsvorgang unterzogen oder direkt abgepackt werden.

### Bezugszeichenliste

- 10:
- 11:
- 12:
- 13: zylindrischer Formkörper
- 14:
- 15: umlaufendes Förderband
- 16: (beheizbarer) Draht
- 17: Drahthalter
- 18:
- 19:
- 20:
- 21:
- 22: keisbogenförmiger Mitnehmer
- 23: stehende Walze
- 24: quaderförmige Backe
- 25: konkave Ausnehmung
- 26: ruhende Platte

## Patentansprüche

1. Verfahren zur Herstellung von festen Arzneiformen durch Vermischen und Aufschmelzen von mindestens einem pharmakologisch akzeptablen polymeren Bindemittel, mindestens einem pharmazeutischen Wirkstoff und gegebenenfalls üblichen pharmazeutischen Additiven in Abwesenheit eines Lösungsmittels zu einem plastischen Gemisch und Ausformen des Gemisches zu der gewünschten Arzneiform durch Extrusion,
**dadurch gekennzeichnet,**
daß das Ausformen in zwei Schritten erfolgt, wobei man in einem ersten Schritt den extrudierten Strang zu Formkörpern zerteilt und diese Formkörper in einem zweiten Schritt in plastischem Zustand arrondiert.

2. Verfahren nach Anspruch 1, dadurch gekennzeichnet, daß das plastische Gemisch als Strang mit kreisförmigem Querschnitt extrudiert wird.

3. Verfahren nach Anspruch 2, dadurch gekennzeichnet, daß die Formkörper Zylinder mit einem Länge/Durchmesser-Verhältnis von mehr als 1 sind.

4. Verfahren nach einem der Ansprüche 1 bis 3, dadurch gekennzeichnet, daß das Zerteilen des plastischen Gemisches durch Schneiden erfolgt.

5. Verfahren nach einem der Ansprüche 1 bis 4, dadurch gekennzeichnet, daß das Arrondieren durch Kontakt zwischen dem Formkörper und mindestens einem gegebenenfalls beheizten Arrondierwerkzeug erfolgt.

6. Verfahren nach Anspruch 5, dadurch gekennnzeichnet, daß der Formkörper und/oder das Arrondierwerkzeug bewegt wird.

7. Verfahren nach einem der Ansprüche 1 bis 6, dadurch gekennzeichnet, daß das Gemisch zu Oblongtabletten ausgeformt wird.
